# EUROPÄISCHE PATENTSCHRIFT

(11) **EP 3 941 201 B1**
(45) Veröffentlichungstag und Bekanntmachung des Hinweises auf die Patenterteilung: **19.07.2023**
(21) Anmeldenummer: 20708034.2
(22) Anmeldetag: 14.02.2020
(51) Int. Cl.: A01N 43/80, A01N 25/04, A01P 1/00, C07D 275/06

(54) **WÄSSRIGE 1,2-BENZISOTHIAZOLIN-3-ON-DISPERSIONEN**
AQUEOUS 1,2-BENZISOTHIAZOLINE-3-ONE DISPERSIONS
DISPERSIONS AQUEUSES DE 1,2-BENZISOTHIAZOLIN-3-ONE

(30) Priorität: 19.03.2019 EP 19000134
(43) Veröffentlichungstag der Anmeldung: 26.01.2022
(73) Patentinhaber: THOR GmbH, 67346 Speyer (DE)
(72) Erfinder: BAUM, Rüdiger, 68809 Neulussheim (DE)
(86) Internationale Anmeldenummer: PCT/EP2020/000042
(87) Internationale Veröffentlichungsnummer: WO 2020/187440

(56) Entgegenhaltungen:
- WO-A1-94/16564
- WO-A1-2012/158425
- US-A- 4 188 376

## Beschreibung

Die Erfindung betrifft wässrige Benzisothiazolin-3-on-Dispersionen von in Wasser dispergierten Natrium-1,2-Benzisothiazolin-3-on-Partikeln. Die Dispersionen enthalten: (a) 15 Gew.-% bis 35 Gew.-% Natrium-1,2-Benzisothiazolin-3-on und (b) ad. 100 Gew.-% Wasser, mit einem Gehalt an Natriumionen im Bereich von 4 bis 10 Gew.-%, bezogen auf das in der Dispersion enthaltene Wasser. Die Erfindung betrifft ferner die Verwendung der erfindungsgemäßen Dispersionen zur Konservierung von wasserbasierten Produkten aller Art, insbesondere von Reinigungs- und Haushaltsprodukten, Kunststoffdispersionen, Farben, Putzen, Klebstoffen, Dichtungsmassen, Papierbeschichtungsmassen, Textilweichmacher- und Schlichtungsmitteln, Waschrohstoffen, Tensiden, Poliermitteln, Spinnbädern, Kühlschmierstoffen, Lederbehandlungsmitteln und Silikon- und Bitumenemulsionen.

Das 1,2-Benzisothiazolin-3-on (BIT) bzw. dessen Natrium-, Kalium- und Lithiumsalze ist ein seit langem in der Praxis verwendeter Wirkstoff zur Herstellung mikrobizid wirksamer Formulierungen. Der Wirkstoff zeichnet sich aus über eine gute chemische und thermische Stabilität, und verfügt grundsätzlich über eine breite antimikrobielle Wirkung gegenüber Bakterien, Pilzen und Hefen.

Um diesen Wirkstoff in leicht handhabbarer und gut dosierbarer Form zur Verfügung zu stellen, werden aus dem bei der Synthese anfallenden rohen BIT zumeist wässrige Lösungen der Alkalimetallsalze des BIT oder wässrige Dispersionen, in denen das BIT in partikulärer Form in der Wasserphase dispergiert ist, hergestellt. Die wässrigen Lösungen sind jedoch auf BIT-Gehalte von unter 10 Gew.-% BIT beschränkt. Höher konzentrierte Lösungen neigen bei Temperaturen unter 20°C zur Kristallisation und sind daher nicht ausreichend lagerstabil.

Zur Herstellung vermeintlich stabiler wässriger BIT-Lösungen in höherer Konzentration als 10 %, werden gemäß der Lehre der DE 28 40 273 A1, dem BIT in Form seines in Wasser gelösten Alkalisalzes gewisse Hydroxylgruppen enthaltende organische Lösungsmittel, wie Propylenglykol, zugesetzt. Der Zusatz derartiger Lösungsmittel führt jedoch zur Verteuerung der Lösungen und ist auch aus ökologischen Gesichtspunkten unerwünscht.

Weiterhin offenbart die U.S. Patentschrift U.S. 4,871,754, wässrige Formulierungen des Lithiumsalzes des 1,2-Benzisothiazolin-3-ons. Diese Zubereitungen sind bei höheren BIT-Konzentrationen jedoch nicht hinreichend kältestabil, und erfordern ebenfalls den Zusatz eines wasser-mischbaren organischen Lösemittels, wie 1,2 Propylenglykol, was mit den vorgenannten Nachteilen verbunden ist.

Daher ist es vorteilhaft, auf möglichst hoch konzentrierte wässrige Lösungen von 1,2-Benzisothiazolin-3-on, die frei von Lösungsmitteln auf Glykolbasis sind, und sich rasch im Rahmen der Anwendung lösen, zurückgreifen zu können.

Die PCT-Offenlegungsschrift WO 2012/158425 A1 offenbart hoch konzentrierte wässrige Lösungen von 1,2-Benzisothiazolin-3-on, die etwa 0,1 bis etwa 30 Gew.-% 1,2-Benzisothiazolin-3-on, etwa 5 bis 15 Gew.-% Alkalimetallsalz, etwa 50 bis etwa 85 Gew.- % Wasser und etwa 0,1 bis etwa 5 Gew.-%. Chelatbildner enthalten. Beim Nachstellen der Zusammensetzungen gemäß dieser Druckschrift hat sich jedoch herausgestellt, dass diese insbesondere bei Temperaturen unterhalb 20°C nicht hinreichend lagerstabil sind.

Weiterhin sind wässrige Dispersionen von 1,2-Benzisothiazolin-3-on mit einem Gehalt an 1,2-Benzisothiazolin-3-on im Bereich von etwa 20 Gew.-% kommerziell erhältlich. Diese Zusammensetzungen lösen sich in der Anwendung jedoch vergleichsweise langsam in dem zu konservierenden Produkt, was im Rahmen einiger Anwendungen dazu führt, dass diese Partikel abfiltriert werden und damit nicht länger als Wirkstoff zur Verfügung stehen. Dispersionen der Alkalimetallsalze des BIT sind kommerziell nicht erhältlich, da sie zur Bildung größerer Partikel bzw. Kristalle neigen und kein homogenes, lagerfähiges Produkt erhalten wird.

Ausgehend von dem Stand der Technik ist es Aufgabe der Erfindung, gegenüber den aus dem Stand der Technik bekannten Zusammensetzungen, stabilere, 1,2-Benzisothiazolin-3-on-Dispersionen mit einem möglichst hohen Gehalt an 1,2-Benzisothiazolin-3-on zur Verfügung zu stellen. Eine weitere Aufgabe der Erfindung besteht darin, möglichst konzentrierte stabile 1,2-Benzisothiazolin-3-on-Dispersionen bereitzustellen, die sich bei der Anwendung als Biozid im Vergleich zu den aus dem Stand der Technik bekannten Dispersionen, rascher in dem zu konservierenden Produkt lösen.

Gelöst wird diese Aufgabe durch eine wässrige Benzisothiazolin-3-on-Dispersion von in Wasser dispergierten Natrium-1,2-Benzisothiazolin-3-on-Partikeln, enthaltend:
(a) 15 Gew.-% bis 35 Gew.-%, bevorzugt 20 Gew.-% bis 30 Gew.-% Natrium-1,2-Benzisothiazolin-3-on und
(b) ad. 100 Gew.-% Wasser, mit einem Gehalt an Natriumionen im Bereich von 4 bis 10 Gew.-%, bezogen auf das in der Dispersion enthaltene Wasser.

Überraschenderweise werden im Rahmen der vorliegenden Erfindung neue, gegenüber den aus dem Stand der Technik bekannten 1,2-Benzisothiazolin-3-on-Dispersionen (BIT-Dispersionen) lagerstabilere, wässrige Dispersionen zur Verfügung gestellt, die die eingangs beschriebenen Nachteile des Standes der Technik vorteilhafterweise überwinden. Lagerstabil bedeutet dabei, dass die erfindungsgemäßen Dispersionen auch bei längeren Lagerungszeiten nahezu kein Partikelwachstum bzw. Kristallwachstum zeigen. Insbesondere werden im Rahmen der vorliegenden Erfindung wässrige BIT-Dispersionen zur Verfügung gestellt, die sich im Vergleich zu den aus dem Stand der Technik bekannten Dispersionen im Rahmen der Anwendung als Biozid deutlich rascher in der Wasserphase der zu konservierenden Produkte lösen.

Die erfindungsgemäße BIT-Dispersion zeichnet sich dadurch aus, dass sie in einer Wasserphase dispergierte Partikel von Natrium-1,2-Benzisothiazolin-3-on (Na-BIT) enthält. Die Na-BIT-Partikel weisen dabei eine mittlere Partikelgröße (d50) von unter 200 µm. Bevorzugt weisen die in der Wasserphase dispergierten Na-BIT-Partikel eine mittlere Partikelgröße (d50) im Bereich von 10 bis 40 µm auf.

Die vorstehend beschriebenen Partikel des Natriumsalzes des 1,2-Benzisothiazolin-3-ons bestehen im Wesentlichen aus dem Natriumsalz des 1,2-Benzisothiazolin-3-ons, so dass neben dem Natriumsalz des 1,2-Benzisothiazolin-3-ons noch weitere BIT Salze in geringer Menge in den Partikeln enthalten sein können.

Als Komponente (a) enthält die erfindungsgemäße BIT-Dispersion 15 Gew.-% bis 35 Gew.-%, bevorzugt 20 Gew.-% bis 30 Gew.-%, besonders bevorzugt 22 Gew.-% bis 29 Gew.-% Natrium-1,2-Benzisothiazolin-3-on, jeweils bezogen auf das Gesamtgewicht der erfindungsgemäßen BIT-Dispersion.

Als Komponente (b) enthält die erfindungsgemäße BIT-Dispersion ad. 100 Gew.-% Wasser, mit einem Gehalt an Natriumionen im Bereich von 4 bis 10 Gew.-%, bevorzugt 5 Gew.-% bis 9 Gew.-%, besonders bevorzugt 6 Gew.-% bis 8 Gew.-%, jeweils bezogen auf das in der Dispersion enthaltene Wasser, bzw. die Wasserphase der Dispersion. "Gehalt an Natriumionen" bedeutet im Rahmen der Erfindung die Menge an Natriumionen, die in der Wasserphase der erfindungsgemäßen Dispersion als gelöste Natriumionen nachweisbar sind. Die nicht in der Wasserphase gelösten Natriumionen des Natriumsalzes des 1,2-Benzisothiazolin-3-ons werden dabei nicht berücksichtigt.

Der Gehalt an Natriumionen in der Wasserphase der Dispersion innerhalb der vorstehend angegebenen Grenzen lässt sich durch Zugabe eines Natriumionen-enthaltenden Salzes, und auch durch die Verwendung eines Überschusses an Natronlauge bei der Neutralisation des BITs einstellen. Als Quelle für die Natriumionen können prinzipiell alle Natriumionen-enthaltenden Salze oder Mischungen verwendet werden, die bei den gewünschten Lagertemperaturen der erfindungsgemäßen Dispersion eine hinreichende Löslichkeit besitzen und nicht mit dem Natriumsalz des Benzisothiazolinons reagieren. Bevorzugt sind die Salze ausgewählt aus der Gruppe bestehend aus Natriumchlorid, Natriumnitrat, Natriumcarbonat, Natriumacetat, Natriumcitrat, Natriumlactat und Natriumformiat, oder Mischungen dieser. Gemäß einer besonders bevorzugten Ausführungsform der Erfindung wird als Natriumionen-enthaltendes Salz Natriumchlorid verwendet. Gemäß einer alternativ bevorzugten Ausführungsform der Erfindung enthalten die erfindungegemäßen Dispersionen neben den vorstehend genannten Natriumionen-enthaltenden Salzen auch Natriumsalze biozider Wirkstoffe, wie Natriumsalicylat, Natriumbenzoat und Natriumpyrithion oder Mischungen dieser. Die Gegenionen der Natriumionen-enthaltenden Salze, wie die Chloridionen werden nachfolgend auch als die Gegenionen der Natriumionenquelle bezeichnet werden.

Der Gehalt an Natriumionen in der Wasserphase der Dispersionen bewirkt in vorteilhafter Weise, dass die in der erfindungsgemäßen Dispersion enthaltenen Partikel des Natriumsalzes des 1,2-Benzisothiazolin-3-ons als solche in dispergiertem Zustand verbleiben, und nur in geringem Maße mit der Wasserphase in Wechselwirkung treten. Hierdurch wird in vorteilhafter Weise erreicht, dass die mittlere Partikelgröße der Natrium-1,2-Benzisothiazolin-3-onpartikel während der Lagerung der Dispersion nahezu konstant bleibt und ein Wachstum der Partikel zumindest weitgehend vermieden wird.

Der Gehalt an Wasser, in Bezug auf das Gesamtgewicht der erfindungsgemäßen BIT-Dispersion liegt allgemein im Bereich von 40 Gew.-% bis 80 Gew.-%, bevorzugt im Bereich von 50 Gew.-% bis 75 Gew.-%, besonders bevorzugt im Bereich von 60 Gew.-% bis 70 Gew.-%, jeweils bezogen auf das Gesamtgewicht der erfindungsgemäßen BIT-Dispersion.

Gemäß einer bevorzugten Ausführungsform der Erfindung enthalten die wässrigen Dispersionen neben den Komponenten (a) und (b), als eine weitere Komponente (c) 0,1 Gew.-% bis 1,0 Gew.-%, bevorzugt 0,2 Gew.-% bis 0,4 Gew.-%, jeweils bezogen auf das Gesamtgewicht der erfindungsgemäßen BIT-Dispersion, wenigstens eines verdickend wirkenden Stoffes. Die Anwesenheit des verdickend wirkenden Stoffes, der Komponente (c), bewirkt eine zusätzliche Stabilisierung der erfindungsgemäßen Dispersionen und vermeidet die Sedimentation.

Der verdickend wirkende Stoff ist bevorzugt ausgewählt aus der Gruppe, bestehend aus Xanthan-Gum, Carageenan, Alginaten, Tylosen, Carboxymethylcellulose, mikrokristalliner Zellulose, Hydroxyethylcellulose und/oder Polyacrylaten. Gemäß einer besonders bevorzugten Ausführungsform der Erfindung ist der als Komponente (c) enthaltene und verdickend wirkende Stoff Xanthan.

Erfindungsgemäß ist die Biozidzusammensetzung dadurch gekennzeichnet, dass diese einen pH-Wert im Bereich von pH 8 bis pH 12, bevorzugt im Bereich von pH 8,0 bis pH 10 aufweist

Gemäß einer bevorzugten Ausführungsform der Erfindung ist die erfindungsgemäße Dispersion im Wesentlichen frei von flüchtigen organischen Verbindungen (VOC), Glykolen, Derivaten von Glykolen, wie 1,2-Propandiol, Glyzerin und / oder Derivaten von Glycerin. "Im Wesentlichen frei" bedeutet in diesem Zusammenhang, dass die Dispersion 0 bis 5 Gew.-%, bevorzugt 0 bis 2 Gew.-%, besonders bevorzugt 0 bis 0,5 Gew.-% flüchtige organische Verbindungen (VOC), Glykole, Derivate von Glykolen, Glyzerin und / oder Derivate von Glycerin enthält. Gemäß einer besonders bevorzugten Ausführungsform der Erfindung, ist die erfindungsgemäße Dispersion frei von flüchtigen organischen Verbindungen (VOC), Glykolen, Derivaten von Glykolen, Glyzerin und / oder Derivaten von Glycerin.

Die erfindungsgemäßen Dispersionen enthalten, falls notwendig, neben den Komponenten (a), (b) und gegebenenfalls der Komponente (c) gegebenenfalls weitere Komponenten, wie Entschäumer, Netzmittel, Dispergiermittel und Schutzkolloide. Diese sind dem Fachmann bekannt und sind je nach Bedarf in entsprechender Menge in der Dispersion enthalten.

Eine Dispersion, enthaltend ausschließlich die Komponenten (a), (b), die Gegenionen der Natriumionenquelle, bevorzugt die Chloridionen, und gegebenenfalls die Komponente (c) in den angegebenen Gewichtsverhältnissen, ohne Vorliegen einer weiteren mikrobiziden Wirkkomponente, wird im Rahmen der vorliegenden Erfindung als "erfindungsgemäße Dispersion" bezeichnet. Die "erfindungsgemäße Dispersion" kann neben den Komponenten (a), (b), den Gegenionen der Natriumionenquelle und gegebenenfalls der Komponente (c) in den angegebenen Gewichtsverhältnissen ein oder mehrere weitere Bestandteile aufweisen. Der oder die weiteren Bestandteile können dabei eine mikrobizide Wirkung aufweisen, oder sie können keine mikrobizide Wirkung aufweisen, also etwa ein Lösungsmittel, Dispergiermittel oder Suspensionsmittel sein.

In einer weiteren Ausführungsform besteht die "erfindungsgemäße Dispersion" aus den Komponenten (a), (b), den Gegenionen der Natriumionenquelle und gegebenenfalls der Komponente (c) in den oben angegebenen Mengenverhältnissen (also der erfindungsgemäßen Dispersion). Dies bedeutet, dass die erfindungsgemäße Dispersion ausschließlich die Komponenten (a), (b), die Gegenionen der Natriumionenquelle und gegebenenfalls die Komponente (c) enthält.

In einer weiteren Ausführungsform der Erfindung besteht die erfindungsgemäße Dispersion "im Wesentlichen" aus den erfindungsgemäßen Komponenten (a), (b), den Gegenionen der Natriumionenquelle und gegebenenfalls der Komponente (c) d.h., dass neben diesen wohl noch ein oder auch mehrere andere Komponenten enthalten sein können, diese aber nur in sehr geringer Menge enthalten sind.

Die Erfindung betrifft ferner die Verwendung der vorstehend definierten Dispersionen zur Konservierung von wasserhaltigen, oder wasserverdünnbaren technischen Produkten vor Befall und/oder Zerstörung durch Mikroorganismen. Bevorzugt können mit den erfindungsgemäßen Dispersionen funktionelle Flüssigkeiten und wasserhaltige technische Produkte, die anfällig sind gegenüber mikrobiellem Befall, konserviert werden. Im Rahmen der Verwendung zeichnet sich die erfindungsgemäße Dispersion dadurch aus, dass sie sich im Rahmen der bestimmungsgemäßen Verwendung im Vergleich zu den kommerziell erhältlichen Dispersionen deutlich schneller in der Wasserphase der zu konservierenden wasserhaltigen oder wasserverdünnbaren technischen Produkten löst.

Die Verwendung der erfindungsgemäßen Dispersionen ist aufgrund des breiten Wirkspektrums des BIT besonders geeignet für die Konservierung von Reinigungs- und Haushaltsprodukten, Kunststoffdispersionen, Farben, Putzen, Klebstoffen, Dichtungsmassen, Papierbeschichtungsmassen, Textilweichmacher- und Schlichtungsmitteln, Waschrohstoffen, Tensiden, Poliermitteln, Spinnbädern, Kühlschmierstoffen, Lederbehandlungsmitteln und Silikon- und Bitumenemulsionen. Besonders bevorzugt eignen sich die erfindungsgemäßen Dispersionen für die Konservierung von Reinigungs- und Haushaltsprodukten sowie Kunststoffdispersionen und Farben.

Die Anwendungskonzentrationen der erfindungsgemäß zu verwendenden Dispersionen richten sich nach der Art und dem Vorkommen der zu bekämpfenden Mikroorganismen, der mikrobiellen Ausgangsbelastung sowie nach der Zusammensetzung des zu schützenden technischen Produkts bzw. Materials. Die optimale Einsatzmenge für eine bestimmte Anwendung kann vor dem Praxiseinsatz durch Testreihen im Labor ermittelt werden. Im Allgemeinen liegen die Anwendungskonzentrationen im Bereich von 0,01 bis 5 Gew.-%, vorzugsweise von 0,02 bis 0,5 Gew.-% der erfindungsgemäßen Dispersion, bezogen auf das zu schützende technische Produkt, bzw. Material.

Die Erfindung betrifft daher gemäß einer Ausführungsform auch ein wasserhaltiges technisches Produkt, ausgewählt aus Reinigungs- und Haushaltsprodukten, Kunststoffdispersionen, Farben, Putzen, Klebstoffen, Dichtungsmassen, Papierbeschichtungsmassen, Textilweichmacher- und Schlichtungsmitteln, Waschrohstoffen, Tensiden, Poliermitteln, Spinnbädern, Kühlschmierstoffen, Lederbehandlungsmitteln und Silikon- und Bitumenemulsionen, das die erfindungsgemäße Dispersion, bevorzugt in einer Menge im Bereich von 0,01 bis 5 Gew.-%, vorzugsweise von 0,02 bis 0,5 Gew.-%, bezogen auf das zu schützende technische Produkt, enthält.

Die Erfindung betrifft ferner ein Verfahren zum Kontrollieren des Wachstums mindestens eines Mikroorganismus in einer wässrigen Flüssigkeit, umfassend den Schritt des Zugebens einer wässrigen Benzisothiazolin-3-on-Dispersion von in Wasser dispergierten Natrium-1,2-Benzisothiazolin-3-on-Partikeln, enthaltend:
(a) 15 Gew.-% bis 35 Gew.-% Natrium-1,2-Benzisothiazolin-3-on und
(b) ad. 100 Gew.-% Wasser, mit einem Gehalt an Natriumionen im Bereich von 4 bis 10 Gew.-%, bezogen auf das in der Dispersion enthaltene Wasser, zu einer wässrigen Flüssigkeit, so dass das Wachstum des wenigstens einen Mikroorganismus in der Flüssigkeit inhibiert, oder wenigstens gehemmt ist. Gemäß einer Ausführungsform der Erfindung ist die wässrige Flüssigkeit ein Wasch- oder Reinigungsmittel, oder eine Polymerdispersion, oder eine Dispersionsfarbe.

Im Rahmen der Verwendung der erfindungsgemäßen Dispersion bei der Durchführung des vorstehend beschriebenen Verfahrens zur Konservierung von wässrigen Produkten bzw. zum Kontrollieren des Wachstums mindestens eines Mikroorganismus in einer wässrigen Flüssigkeit hat sich herausgestellt, dass sich die erfindungsgemäße Dispersion im Vergleich zu den kommerziell erhältlichen Dispersionen deutlich schneller in der Wasserphase der zu konservierenden wässrigen Flüssigkeit löst. Hierdurch können beispielsweise erheblich verkürzte Einarbeitungszeiten bzw. Rührzeiten realisiert werden. Ein weiterer Vorteil bei der Verwendung der erfindungsgemäßen Dispersionen zur Konservierung von Polymerdispersionen, oder Dispersionsfarben liegt darin, dass aufgrund der verbesserten Löslichkeit der erfindungsgemäßen Dispersion in der Wasserphase dieser Produkte, die enthaltenen Natrium-1,2-Benzisothiazolin-3-on-Partikel in deutlich geringerem Maße in der Polymermatrix der Polymerdispersionen beziehungsweise der Dispersionsfarben gebunden werden, und daher auch in geringerem Maße mit eventuell in der Matrix noch vorhandenen Polymerisationskatalysatoren reagieren können.

Die erfindungsgemäßen Dispersionen können beispielsweise dadurch hergestellt werden, indem man Wasser und Natriumhydroxid vorlegt und das 1,2-Benzisothiazolin-3-on, vorzugsweise in Form eines feuchten Filterkuchens, unter Rühren bei einer Temperatur von etwa 40 bis 60 °C in Wasser zugibt und das erhaltene Gemisch bei einer Temperatur von etwa 40 bis 60 °C rührt, bis sich der Feststoff aufgelöst hat. Daran anschließend wird die entsprechende Menge an Natriumchlorid hinzugegeben und die Zusammensetzung auf Raumtemperatur abgekühlt. Formulierungshilfsmittel werden, je nach Art, mit dem Wasser zusammen vorgelegt, oder am Ende zugefügt. In einem nächsten Verfahrensschritt werden die ausgefallenen Natrium-1,2-Benzisothiazolin-3-on-Partikel bis zur gewünschten Partikelgröße, bevorzugt bis zu einer Partikelgröße nach Hegman von kleiner < 60 µm, vermahlen.

Gemäß eines alternativen Herstellungsverfahrens wurde zunächst eine bei 50°C annäherend gesättigte BIT Na Lösung hergestellt und diese Lösung, unter kräftigem Rühren am Dissolver, in eine Vorlage aus Wasser, Schutzkolloid und Natriumchlorid eingerührt. Die dabei entstandene BIT Na Dispersion wurde mit Xanthan stabilisiert und wies eine Partikelgröße nach Hegman von kleiner 200 µm auf.

Gemäß einer bevorzugten Ausführungsform der Erfindung enthält die erfindungsgemäße Dispersion die folgenden Komponenten, beziehungsweise besteht aus den folgenden Komponenten:
Zusammensetzung 1:

| | |
|---|---|
| 1,2-Benzisothiazolin-3-on, Natriumsalz | 23 Gew.-% |
| NaCl | 12 Gew.-% |
| Wasser | 64,7 Gew.-% |
| Xanthan gum | 0,3 Gew.-% |

Zusammensetzung 2:

| | |
|---|---|
| 1,2-Benzisothiazolin-3-on, Natriumsalz | 29 Gew.-% |
| NaCl | 10 Gew.-% |
| Wasser | 60,7 Gew.-% |
| Xanthan gum | 0,3 Gew.-% |

**Die folgenden Beispiele und Vergleichsbeispiele dienen zur weiteren Veranschaulichung der vorliegenden Erfindung.**

Zur Untersuchung der Stabilität der erfindungsgemäßen Dispersionen wurden die in der nachfolgend dargestellten Tabelle 1 definierten Dispersionen hergestellt. Die mittlere Partikelgröße der Natrium-1,2-Benzisothiazolin-3-on-Partikel lag im Bereich von 10 bis 40 µm. Bei den Dispersionen 1, 2, 3, 5 und 6 handelt es sich um erfindungsgemäße Dispersionen, die Dispersionen 4 C und C7 dienen zum Vergleich.

Zur Untersuchung der Lagerstabilität der jeweiligen Dispersionen wurden die Dispersionen in 5 Zyklen jeweils 16 h bei 40°C gelagert und anschließend 8 h bei 20°C abkühlen gelassen.

Wie man den in der Tabelle 1 dargestellten Ergebnissen entnehmen kann, ließ sich in den Beispielen 1, 2, 3, 5 und 6 kein Wachstum der Natrium-1,2-Benzisothiazolin-3-on-Partikel beobachten, wohingegen in den Vergleichsbeispielen 4 C und 7 C eine ausgeprägte Kristallbildung zu beobachten war.

**Tabelle 1:**

| | Beispiele 1, 2, 3, 5 und 6 und Vergleichsbeispiele 4 C und 7 C | | | | | | |
|---|---|---|---|---|---|---|---|
| | 1 | 2 | 3 | 4C | 5 | 6 | 7C |
| Komponenten | Gew.-% | Gew.-% | Gew.-% | Gew.-% | Gew.-% | Gew.-% | Gew.-% |
| BIT nominal | 20 | 25 | 20 | 20 | 15 | 30 | 20 |
| BIT 85 %ig | 23,5 | 29,4 | 23,5 | 23,5 | 17,7 | 35,3 | 23,5 |
| NaOH für NaBIT | 5,3 | 6,6 | 5,3 | 5,3 | 4 | 8 | 5,3 |
| NaOH Überschuss | 0 | 1 | 0 | 0 | 0 | 0 | 0 |
| NaCl | 15 | 12,5 | 10 | 5 | 15 | 15 | 0 |
| Xanthan | 0,3 | 0,25 | 0,3 | 0,3 | 0,3 | 0,2 | 0,3 |
| Wasser | 55,9 | 50,25 | 60,9 | 65,9 | 63 | 41,5 | 70,9 |
| | | | | | | | |
| Wassergehalt (gesamt) | 61,8 | 57,6 | 66,8 | 71,8 | 67,4 | 50,4 | 76,8 |
| | | | | | | | |
| gelöstes Na⁺/ in % pro Wasserphase* | 7,7 | 7,7 | 5,1 | 2,6 | 7,2 | 9,0 | 0,0 |
| | | | | | | | |
| NaCl in der Wasserphase | 19,5 | 17,6 | 13,0 | 6,5 | 18,2 | 22,9 | 0,0 |
| | | | | | | | |
| Stabilität nach fünfmaligen Erhitzen auf 40°C und Abkühlen auf 20°C | OK | OK | OK | Kristallbildung | OK | OK | ausgeprägte Kristallbildung |

| | | | | | | | |
|---|---|---|---|---|---|---|---|
| * Wasserphase = Wasser plus NaCl plus NaOH Überschuss | | | | | | | |

## Patentansprüche

1. Wässrige Benzisothiazolin-3-on-Dispersion von in Wasser dispergierten Natrium-1,2-Benzisothiazolin-3-on-Partikeln, enthaltend:
(a) 15 Gew.-% bis 35 Gew.-% Natrium-1,2-Benzisothiazolin-3-on und
(b) ad. 100 Gew.-% Wasser, mit einem Gehalt an Natriumionen im Bereich von 4 bis 10 Gew.-%, bezogen auf das in der Dispersion enthaltene Wasser und ohne Berücksichtigung der nicht in der Wasserphase gelösten Natriumionen des Natriumsalzes des 1,2-Benzisothiazolin-3-ons.

2. Dispersion nach Anspruch 1, enthaltend als weitere Komponente:
(c) 0,1 bis 1 Gew.-% wenigstens eines verdickend wirkenden Stoffes.

3. Dispersion nach Anspruch 2, **dadurch gekennzeichnet, dass** der verdickend wirkende Stoff ausgewählt ist aus der Gruppe, bestehend aus Xanthan-Gum, Carageenan, Alginaten, Tylosen, Carboxymethylcellulose, mikrokristalliner Zellulose, Hydroxyethylcellulose und/oder Polyacrylaten.

4. Dispersion nach einem der Ansprüche 1 bis 3, **dadurch gekennzeichnet, dass** die Dispersion das Natrium-1,2-Benzisothiazolin-3-on in Form von Partikeln mit einer mittleren Partikelgröße im Bereich von 10 bis 40 µm enthält.

5. Dispersion nach einem der Ansprüche 1 bis 4, **dadurch gekennzeichnet, dass** die Dispersion als Komponente (a) 20 Gew.-% bis 30 Gew.-% Natrium-1,2-Benzisothiazolin-3-on enthält.

6. Dispersion nach einem der Ansprüche 1 bis 5, **dadurch gekennzeichnet, dass** die Dispersion einen pH-Wert im Bereich von pH 8 bis pH 12 aufweist.

7. Dispersion nach einem der Ansprüche 1 bis 6, **dadurch gekennzeichnet, dass** die Dispersion im Wesentlichen frei von flüchtigen organischen Verbindungen (VOC), Glykolen, Derivaten von Glykolen, Glyzerin und / oder Derivaten von Glycerin ist.

8. Verwendung einer Dispersion gemäß einem der Ansprüche 1 bis 7 als Konservierungsmittel zum Schutz von wasserhaltigen technischen Produkten vor Befall und/oder Zerstörung durch Mikroorganismen.

9. Verwendung nach Anspruch 8, **dadurch gekennzeichnet, dass** das wasserhaltige technische Produkt ausgewählt ist aus Reinigungs- und Haushaltsprodukten, Kunststoffdispersionen, Farben, Putzen, Klebstoffen, Dichtungsmassen, Papierbeschichtungsmassen, Textilweichmacher- und Schlichtungsmitteln, Waschrohstoffen, Tensiden, Poliermitteln, Spinnbädern, Kühlschmierstoffen, Lederbehandlungsmitteln und Silikon- und Bitumenemulsionen.

10. Wasserhaltiges technisches Produkt, ausgewählt aus Reinigungs- und Haushaltsprodukten, Kunststoffdispersionen, Farben, Putzen, Klebstoffen, Dichtungsmassen, Papierbeschichtungsmassen, Textilweichmacher- und Schlichtungsmittel, Waschrohstoffen, Tensiden, Poliermitteln, Spinnbädern, Kühlschmierstoffen, Lederbehandlungsmitteln und Silikon- und Bitumenemulsionen, enthaltend eine Dispersion nach einem der Ansprüche 1 bis 7.

11. Wasserhaltiges technisches Produkt nach Anspruch 10, enthaltend eine Dispersion nach einem der Ansprüche 1 bis 7 in einem Anteil von 0,01 bis 5 Gew.-%.

12. Verfahren zum Kontrollieren des Wachstums mindestens eines Mikroorganismus in einer wässrigen Flüssigkeit, umfassend den Schritt des Zugebens einer wässrigen Benzisothiazolin-3-on-Dispersion von in Wasser dispergierten Natrium-1,2-Benzisothiazolin-3-on-Partikeln, enthaltend:
(a) 15 Gew.-% bis 35 Gew.-% Natrium-1,2-Benzisothiazolin-3-on und
(b) ad. 100 Gew.-% Wasser, mit einem Gehalt an Natriumionen im Bereich von 4 bis 10 Gew.-%, bezogen auf das in der Dispersion enthaltene Wasser und ohne Berücksichtigung der nicht in der Wasserphase gelösten Natriumionen des Natriumsalzes des 1,2-Benzisothiazolin-3-ons,
zu einer wässrigen Flüssigkeit, so dass das Wachstum des wenigstens einen Mikroorganismus in der Flüssigkeit inhibiert, oder wenigstens gehemmt ist.

13. Verfahren nach Anspruch 12, **dadurch gekennzeichnet, dass** die wässrige Flüssigkeit ein Wasch- oder Reinigungsmittel oder eine Polymerdispersion oder eine Dispersionsfarbe ist.

## Claims

1. Aqueous benzisothiazoline-3-one dispersion of sodium-1,2-benzisothiazoline-3-one particles dispersed in water, comprising:
(a) between 15 wt.-% and 35 wt.-% of sodium-1,2-benzisothiazoline-3-one; and
(b) ad. 100 wt.-% of water having a content of sodium ion in the range from 4 wt.- % to 10 wt.-%, with respect to water contained in the dispersion.

2. Dispersion according to claim 1, comprising as further component:
(c) 0.1 wt.-% to 1 wt.-% of at least one thickening agent.

3. Dispersion according to claim 2, **characterized in that** the thickening agent is selected from the group consisting of xanthan gum, carrageenan, alginates, tyloses, carboxymethyl cellulose, microcrystalline cellulose, hydroxyethyl cellulose and/or polyacrylates.

4. Dispersion according to any one of claims 1 to 3, **characterized in that** the dispersion contains the sodium-1,2-benzisothiazoline-3-one in the form of particles having a median particle size in the range from 10 to 40 µm.

5. Dispersion according to any one of claims 1 to 4, wherein the dispersion contains, as component (a), between 20 wt.-% and 30 wt.-% of sodium-1,2-benzisothiazoline-3-one.

6. A dispersion according to any one of claims 1 to 5, **characterized in that** the dispersion has a pH in the range from pH 8 to pH 12.

7. A dispersion according to any one of claims 1 to 6, **characterized in that** the dispersion is substantially free of volatile organic compounds (VOC), glycols, derivatives of glycols, glycerol and/or derivatives of glycerol.

8. Use of a dispersion according to any one of claims 1 to 7 as a preservative for protecting water-containing technical products from attack and/or destruction by microorganisms.

9. Use according to claim 8, **characterized in that** the water-containing technical product is selected from cleaning and household products, plastic dispersions, paints, plasters, adhesives, sealing compounds, paper coating compounds, textile softening and sizing agents, washing raw materials, surfactants, polishing agents, spinning baths, cooling lubricants, leather treatment agents and silicone and bitumen emulsions.

10. Water-containing technical product selected from cleaning and household products, plastics dispersions, paints, plasters, adhesives, sealants, paper coating compounds, textile softeners and sizing agents, washing raw materials, surfactants, polishing agents, spinning baths, cooling lubricants, leather treatment agents, and silicone and bitumen emulsions, comprising a dispersion according to any one of claims 1 to 7.

11. Aqueous technical product according to claim 10, comprising a dispersion according to any one of claims 1 to 7 in a proportion of 0.01 wt.-% to 5 wt.-%.

12. Method for controlling the growth of at least one microorganism in an aqueous liquid comprising the step of adding an aqueous benzisothiazoline-3-one dispersion of water dispersed sodium-1,2-benzisothiazoline-3-one particles containing:
(a) between 15 wt.-% and 35 wt.-% of sodium-1,2-benzisothiazoline-3-one; and
(b) ad. 100 wt.-% of water having a content of sodium ions in the range from 4 wt.- % to 10 wt.-%, with respect to the water contained in the dispersion, to an aqueous liquid, so that the growth of the at least one microorganism in the liquid is inhibited, or at least suppressed.

13. The process according to claim 12, **characterized in that** the aqueous liquid is a detergent or cleaning agent or a polymer dispersion or an emulsion paint.

## Revendications

1. Dispersion aqueuse de benzisothiazolin-3-one de particules de sel de sodium de 1,2-benzisothiazolin-3-one dispersées dans l'eau, contenant :
(a) 15 % en poids à 35 % en poids de sel de sodium de 1,2-benzisothiazolin-3-one et
(b) en complément à 100 % en poids d'eau, comportant une teneur en ions sodium dans la plage de 4 à 10 % en poids, par rapport à l'eau contenue dans la dispersion et sans tenir compte des ions sodium non dissous dans la phase aqueuse du sel de sodium de 1,2-benzisothiazolin-3-one.

2. Dispersion selon la revendication 1, contenant comme composant supplémentaire :
(c) 0,1 à 1 % en poids d'au moins une substance ayant un effet épaississant.

3. Dispersion selon la revendication 2, **caractérisée en ce que** la substance ayant un effet épaississant est choisie dans le groupe, constitué par une gomme xanthane, un carraghénane, des alginates, des tyloses, une carboxyméthylcellulose, une cellulose microcristalline, une hydroxyéthylcellulose et/ou des polyacrylates.

4. Dispersion selon l'une quelconque des revendications 1 à 3, **caractérisée en ce que** la dispersion contient le sel de sodium de 1,2-benzisothiazolin-3-one sous forme de particules dotées d'une taille moyenne de particule dans la plage de 10 à 40 µm.

5. Dispersion selon l'une quelconque des revendications 1 à 4, **caractérisée en ce que** la dispersion contient en tant que composant (a) 20 % en poids à 30 % en poids de sel de sodium de 1,2-benzisothiazolin-3-one.

6. Dispersion selon l'une quelconque des revendications 1 à 5, **caractérisée en ce que** la dispersion présente une valeur de pH dans la plage de pH 8 à pH 12.

7. Dispersion selon l'une quelconque des revendications 1 à 6, **caractérisée en ce que** la dispersion est essentiellement exempte de composés organiques volatils (COV), de glycols, de dérivés de glycols, de glycérine et/ou de dérivés de glycérine.

8. Utilisation d'une dispersion selon l'une quelconque des revendications 1 à 7 en tant qu'agent de conservation pour la protection de produits techniques contenant de l'eau contre une attaque et/ou destruction par des micro-organismes.

9. Utilisation selon la revendication 8, **caractérisée en ce que** le produit technique contenant de l'eau est choisi parmi des produits de nettoyage et des produits ménagers, des dispersions de matière plastique, des peintures, des enduits, des adhésifs, des masses d'étanchéité, des masses de revêtement de papier, des assouplissants pour textile et des colles, des bases lavantes, des tensioactifs, des agents de polissage, des bains de filage, des lubrifiants réfrigérants, des agents de traitement du cuir et des émulsions de silicone et des émulsions de bitume.

10. Produit technique contenant de l'eau, choisi parmi des produits de nettoyage et des produits ménagers, des dispersions de matière plastique, des peintures, des enduits, des adhésifs, des masses d'étanchéité, des masses de revêtement de papier, des assouplissants pour textile et des colles, des bases lavantes, des tensioactifs, des agents de polissage, des bains de filage, des lubrifiants réfrigérants, des agents de traitement du cuir et des émulsions de silicone et des émulsions de bitume, contenant une dispersion selon l'une quelconque des revendications 1 à 7.

11. Produit technique contenant de l'eau selon la revendication 10, contenant une dispersion selon l'une quelconque des revendications 1 à 7 en une proportion de 0,01 à 5 % en poids.

12. Procédé pour la régulation de la croissance d'au moins un micro-organisme dans un liquide aqueux, comprenant l'étape d'ajout d'une dispersion aqueuse de benzisothiazolin-3-one de particules de sel de sodium de 1,2-benzisothiazolin-3-one dispersées dans l'eau, contenant :
(a) 15 % en poids à 35 % en poids de sel de sodium de 1,2-benzisothiazolin-3-one et
(b) en complément à 100 % en poids d'eau, comportant une teneur en ions sodium dans la plage de 4 à 10 % en poids, par rapport à l'eau contenue dans la dispersion et sans tenir compte des ions sodium non dissous dans la phase aqueuse du sel de sodium de 1,2-benzisothiazolin-3-one,
à un liquide aqueux, de sorte que la croissance de l'au moins un micro-organisme dans le liquide est inhibée, ou au moins entravée.

13. Procédé selon la revendication 12, **caractérisé en ce que** le liquide aqueux est un agent de lavage ou un agent de nettoyage ou une dispersion de polymère ou une peinture à dispersion.
